# EUROPEAN PATENT SPECIFICATION

(11) **EP 3 141 895 B1**
(45) Date of publication and mention of the grant of the patent: **09.05.2018**
(21) Application number: 15812869.4
(22) Date of filing: 01.09.2015
(51) Int. Cl.: G01N 31/00, G01N 31/12, G01N 21/27, G01N 21/76, G01N 33/00, G01N 1/38

(54) **NITROGEN ANALYSIS DEVICE**
STICKSTOFFANALYSEVORRICHTUNG
DISPOSITIF D'ANALYSE D'AZOTE

(30) Priority: 16.07.2015 JP 2015141972
(43) Date of publication of application: 15.03.2017
(73) Proprietor: Mitsubishi Chemical Analytech Co., Ltd., Yamato-shi, Kanagawa 2420001 (JP)
(72) Inventor: KAGAWA, Shuji, Chigasaki-shi, Kanagawa 253-0084 (JP); HECHT, Henrik, 40595 Düsseldorf (DE); SATO, Kana, Chigasaki-shi, Kanagawa 253-0084 (JP); TOMOYOSE, Tamaki, Chigasaki-shi, Kanagawa 253-0084 (JP)
(74) Representative: Hoffmann Eitle
(86) International application number: PCT/JP2015/074870
(87) International publication number: WO 2017/010021

(56) References cited:
- JP-A- S6 282 360
- JP-A- H10 274 657
- JP-A- H10 282 083
- JP-A- H10 282 083
- JP-A- H11 352 058
- JP-A- H11 352 058
- JP-A- S55 136 957
- JP-A- 2000 039 385
- JP-A- 2003 164 852
- JP-A- 2008 082 808
- JP-B2- 4 811 221
- CHAWLA BIRBAL: "Speciation of Nitrogen Compounds in Gasoline and Diesel Range Process Streams by Capillary Column Gas Chromatography with Chemiluminescence Detection", JOURNAL OF CHROMATOGRAPHIC SCIENCE, OXFORD UNIVERSITY PRESS, CARY, NC, USA, vol. 35, no. 3, 1997, pages 97-104, XP009086208, ISSN: 0021-9665

## Description

### TECHNICAL FIELD

The present invention relates to a nitrogen analyzer, and more particularly, to a nitrogen analyzer used for practicing a method for quantitative analysis of nitrogen in a specimen by a chemiluminescence method using ozone which is capable of measuring a concentration of nitrogen contained in a specimen such as, for example, petroleum, with still higher accuracy.

### BACKGROUND ART

In the quantitative analysis of nitrogen contained in fuel-related specimens, e.g., diesel fuels, petroleum such as oils and gasoline, and coals such as coal tar, aqueous solution specimens, e.g., environmental waters such as river water and lake water, and industrial waste waters, as well as various polymer material specimens and organic liquid specimens, there has been used a nitrogen analyzer utilizing chemiluminescence measured using ozone. In the nitrogen analysis using such a nitrogen analyzer, a specimen is injected into a reaction tube, and the reaction tube is heated while feeding oxygen thereinto to burn the specimen and recover a specimen gas from the reaction tube, and then the resulting specimen gas is allowed to react with ozone generated in an ozone generator to thereby measure a chemiluminescence intensity due to the reaction using a chemiluminescence detector.

The aforementioned nitrogen analyzing method utilizes such a fact that in the case where nitrogen monoxide (NO) generated upon combustion of a nitrogen-containing compound is reacted with ozone (O₃) generated in an ozone generator to produce nitrogen dioxide (NO₂), the intensity of chemiluminescence generating in the reaction has a correlation with the nitrogen monoxide which is represented by a linear equation (Y = aX + b wherein X is a concentration of nitrogen monoxide or a weight of nitrogen in terms of nitrogen monoxide; Y is a chemiluminescence intensity; and a and b are each a constant coefficient) (refer to Patent Literatures 1 and 2). For example, in the aforementioned nitrogen analysis for the fuel-related specimens, the specimen to be measured is diluted with a solvent to prepare a diluted specimen having an appropriate concentration, and the resulting diluted specimen is subjected to the above analysis procedure in view of measuring ranges of the chemiluminescence detector and handling property of the specimen.

### CITATION LIST:

### PATENT LITERATURE

Patent Literature 1: Japanese Patent No. 4779911
Patent Literature 2: Japanese Patent No. 4811221

JP H10 282083 A describes a method for measuring ammonium ion in water. In the method for measuring ammonium ion in water, the concentration of ammonium ions in sample water in a high-concentration area is measured by using a flow injection method after the sample water is diluted to a low-concentration area by utilizing the flow rate characteristic. For diluting the sample water, a means which dilutes the sample water by mixing the water fed from a constant-flow pump with diluting water fed from another constant-flow pump. Pump rotation controllers and control mechanisms respectively installed to the constant-flow pumps are provided and the flow rates of the sample water and diluting water from the pumps are controlled by selecting a rate of dilution out of the rates of dilution set in the control mechanisms and conveying the selected rate of dilution to the pump rotation controllers.

CHAWLA BIRBAL: "Speciation of Nitrogen Compounds in Gasoline and Diesel Range Process Streams by Capillary Column Gas Chromatography with Chemiluminescence Detection", JOURNAL OF CHROMATOGRAPHIC SCIENCE, OXFORD UNIVERSITY PRESS, CARY, NC, USA, vol. 35, no. 3 (1997), XP009086208, ISSN: 0021-9665. In this literature, there are described a method for qualitative analysis in which among a plurality of peak components detected by gas chromatography, only nitrogen-containing peak components are specifically determined by additional use of a chemiluminescence detector, as well as the results of quantitative analysis for conducting quantitative determination of the nitrogen-containing components using a calibration curve prepared in the range of 0 to 80 ppm.

JP H11 352058 (A) describes a water quality analyzer. The analyzer has a specimen introduction part equipped with a diluting function. In the case where a measured value is deviated from a maximum input value or threshold value in a previously prepared calibration curve, the dilution ratio or sensitivity of the detector, or both of the dilution ratio and sensitivity of the detector are automatically changed to adjust the measured value within the range of the calibration curve for quantitative determination of the specimen.

### SUMMARY OF INVENTION

### TECHNICAL PROBLEM

Meanwhile, the reason why the specimen is diluted as the pretreatment in the above nitrogen analysis, is as follows. That is, when the concentrations of nitrogen in the respective specimens are largely different from each other, the production efficiency of nitrogen monoxide upon combustion of each specimen is also varied. Therefore, even when it is intended to use a calibration curve prepared based on the correlation of linear equation, since the dynamic range thereof tends to be excessively narrow, it is not possible to immediately utilize the calibration curve. For this reason, in the measurement, the specimen is appropriately diluted to such an extent that the concentration of nitrogen in the specimen is reduced to not more than 1000 ppm.

The procedure for diluting the specimen requires skilled delicate technologies using a metering container such as a measuring flask, a whole pipette and a measuring pipette. However, it has been actually confirmed that the analysis accuracy is very low such that the true concentration is inconsistent with the analyzed value. In particular, in the case where the concentration of nitrogen in the specimen after being diluted is more than 1000 ppm, there is such a tendency that deviation of the analyzed value from the true concentration is considerably increased. Furthermore, in the case where the diluting procedure is conducted by a plurality of engineers, even if the same procedure is executed using the same specimen, human errors tend to occur so that the analyzed value tends to be frequently fluctuated.

In addition, in the manual procedure of diluting the fuel-related specimens, it is necessary to avoid and reduce exposure of a human body to carcinogenic substances contained in the specimen. Besides, although the amount of the diluted specimen to be measured is very small, e.g., as small as about 50 *µL*, the diluted specimen tends to be prepared in as large an amount as 20 to 50 mL in the diluting procedure, so that a majority of the thus prepared diluted specimen must be discarded, which results in problems such as large environmental burden.

The present invention has been accomplished in view of the above conventional problems. An object of the present invention is to provide a nitrogen analyzer for practicing a nitrogen analyzing method in which a specimen comprising a nitrogen compound is burned, and the resulting specimen gas is allowed to react with ozone to measure a chemiluminescence intensity thereof, thereby conducting quantitative analysis of nitrogen in the specimen, and which is capable of measuring a concentration of nitrogen contained in the specimen with still higher accuracy. Also, another object of the present invention is to provide a nitrogen analyzer which has a less adverse influence on human body and is also capable of further reducing environmental burden even when analyzing nitrogen in the aforementioned fuel-related specimens.

### SOLUTION TO PROBLEM

In order to solve the above problems, the present inventors first prepared a specimen having a known concentration. The specimen was previously diluted with a solvent to prepare diluted specimens having various different concentrations, and the resulting diluted specimens were subjected to measurement of the chemiluminescence intensity by the aforementioned analyzing method to analyze the relationship between the concentration of nitrogen in the diluted specimen and the chemiluminescence intensity. As a result, it was confirmed that in the case where the concentration of nitrogen in the diluted specimen is in the range of 500 to 2500 ppm, 1000 to 5000 ppm, 3000 to 10000 ppm or 5000 to 20000 ppm, the correlation of linear equation is observed in the respective ranges, but slopes of the respective linear equations are different from each other. In particular, as the concentration of nitrogen in the diluted specimen is increased, the production efficiency of nitrogen monoxide upon combustion tends to be gradually lowered so that the correlation of quadratic equation tends to become more remarkable. Thus, it has been found that in such a case, the analyzed value is more largely deviated from the true value. In the further analysis in which the specimen is diluted until the concentration of nitrogen therein is reduced to not more than 100 ppm, it has been noticed that the relationship between the nitrogen concentration and the chemiluminescence peak area corresponds to a correlation of linear equation whose intercept passes approximately through an origin, i.e., an approximate proportional relation. Thus it has been found that when not only using the curve having the above correlation of linear equation (proportional relation) as a calibration curve, but also diluting even the specimen having a high concentration into the aforementioned low concentration ranges, it is possible to obtain an accurate analyzed value substantially without deviation of the analyzed value from the actual true value irrespective of the concentration of nitrogen in the specimen. The present invention has been accomplished based on the finding.

That is, according to the present invention, there is provided a nitrogen analyzer comprising a specimen gas feeding mechanism, a nitrogen analyzing mechanism and a computer for analysis.

The specimen gas feeding mechanism comprises a reaction tube having a double tube structure comprising an inner tube for introducing a liquid specimen, which comprises at a head portion thereof an automatic syringe, and an outer tube for recovering a specimen gas, the outer tube being configured for feeding oxygen therethrough, and a heating furnace for heating the reaction tube, the specimen gas feeding mechanism having a function of heating and burning the specimen in the reaction tube by the heating furnace to convert nitrogen in the specimen into nitrogen monoxide and recover the nitrogen monoxide as the specimen gas.

The nitrogen analyzing mechanism comprises an ozone generator and a chemiluminescence detector, the chemiluminescence detector being constructed to measure a chemiluminescence intensity based on a reaction between the nitrogen monoxide in the specimen gas fed from the specimen gas feeding mechanism and ozone produced in the ozone generator.

In use, when injecting a predefined amount of the specimen into the specimen gas feeding mechanism, the nitrogen analyzer is configured to perform a diluting procedure in which the specimen is diluted with a solvent to prepare a diluted specimen having a nitrogen concentration of 5 to 100 ppm being conducted in the automatic syringe, and when quantitatively determining a concentration of nitrogen in the specimen from the chemiluminescence intensity obtained in the nitrogen analyzing mechanism, the computer for analysis comprises means for calculating a weight of nitrogen in the diluted specimen based on a calibration curve previously prepared from a standard specimen having a nitrogen concentration of 5 to 100 ppm which expresses a relationship between a chemiluminescence peak area obtained from the chemiluminescence intensity as measured and the weight of nitrogen.

### ADVANTAGEOUS EFFECTS OF INVENTION

In the nitrogen analyzer according to the present invention, by using the diluted specimen prepared by diluting the specimen into a specific concentration range, it is possible to maintain a constant production efficiency of nitrogen monoxide upon burning the specimen, and further since the weight of nitrogen in the specimen can be calculated based on an accurate calibration curve having an approximately proportional relation between the nitrogen concentration and a chemiluminescence peak area in the aforementioned specific concentration range, it is possible to measure concentrations of nitrogen in various specimens that are extremely different in nitrogen concentration from each other, with high accuracy. In addition, by using the automatic syringe to dilute the specimen in the syringe, it is possible to exclude occurrence of human errors in the diluting procedure, and avoid exposure of human body to carcinogenic substances even when analyzing fuel-related specimens. Besides, according to the present invention, it is possible to conduct the analysis using a minimum amount of the diluted specimen and therefore reduce environmental burden.

### BRIEF DESCRIPTION OF DRAWINGS

FIG. 1 is a flow diagram showing a main construction of a nitrogen analyzer used for practicing the nitrogen analyzing method according to the present invention.
FIG. 2 is a sectional view showing a manner of the procedure of diluting the specimen in the nitrogen analyzing method according to the present invention.
FIG. 3 is a graph showing a calibration curve representing a relationship between the chemiluminescence peak area and nitrogen amount obtained in the nitrogen analyzing method according to the present invention.
FIG. 4 is a graph showing a plurality of calibration curves each representing a relationship between the chemiluminescence peak area and nitrogen amount obtained in the conventional analyzing methods.

### DESCRIPTION OF EMBODIMENT

The preferred embodiment of the nitrogen analyzer according to the present invention is explained below by referring to the accompanying drawings. The analyzing method can be applied to various specimens described above, and can be practiced using the nitrogen analyzer utilizing chemiluminescence measured using ozone.

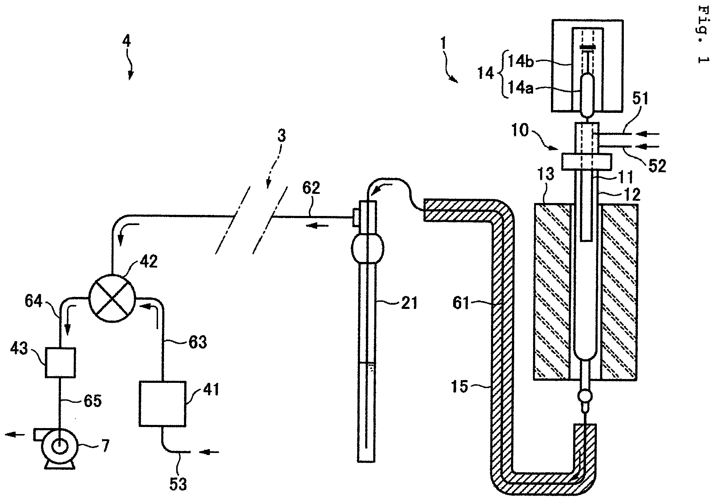

First, an outlined construction of the nitrogen analyzer as an example used in the present invention is described. As shown in FIG. 1, the nitrogen analyzer is constructed mainly of a specimen gas feeding mechanism 1, a nitrogen analyzing mechanism 4 and a computer for analysis (not shown). The specimen gas feeding mechanism 1 comprises a reaction tube 10 into which the specimen is introduced and oxygen is fed, and a heating furnace 13 for heating the reaction tube, and has a function of heating and burning the specimen in the reaction tube 10 by the heating furnace to convert the nitrogen in the specimen into nitrogen monoxide and recover the nitrogen monoxide as the specimen gas.

The reaction tube 10 has a double tube structure comprising an inner tube 11 for introducing the specimen and an outer tube 12 for recovering the specimen gas through which oxygen is fed. The inner tube 11 is provided at a heat portion thereof with an automatic syringe 14 as a specimen injecting device for injecting a liquid specimen. The inner tube 11 is designed so as to have an outer diameter smaller than an inner diameter of the outer tube 12 and a depth shorter than that of the outer tube 12 in order to ensure a clearance for allowing a gas to pass therethrough between an outer peripheral surface of the inner tube 11 and an inner peripheral surface of the outer tube 12. The inner tube 11 is connected at an upper portion thereof with a carrier gas feed passage 51 through which oxygen for promoting burning of the specimen and an inert gas such as argon for transportation are introduced.

On the other hand, the outer tube 12 is constructed of an elongated cylindrical tube that is closed at a bottom end thereof and sealed at an upper end thereof. The outer tube 12 is connected at an upper portion thereof with an oxygen feed passage 52 for introducing oxygen for burning the specimen, and at a bottom thereof with a passage 61 for withdrawing the specimen gas obtained by burning the specimen. In addition, a heater 15 for heating conduits is disposed over an outer periphery of the passage 61 in order to completely oxidize the specimen gas.

The heating furnace 13 serves as a heating means for heating the reaction tube 10, and is constructed of an electric furnace provided at a central portion thereof with a reaction tube insertion hole through which the reaction tube 10 is inserted. More specifically, the heating furnace 13 is constructed of a cylindrical casing, a heat insulator accommodated in the casing and a plurality of heaters embedded in the heat insulator, for example, sheathed heaters produced by accommodating a KANTHAl heating element, a nichrome heating element, a silver heating element or the like in a metal tube. Meanwhile, the heating furnace 13 is designed so as to detect a temperature of the reaction tube 10 and control energization of the heaters based on the temperature thus detected in order to maintain the reaction tube 10 at a predetermined temperature.

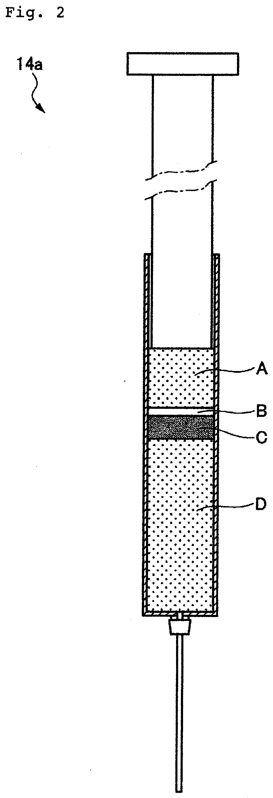

The automatic syringe 14 is constructed of a microsyringe 14a for sampling and injecting the specimen and a plunger pump 14b for fixing the microsyringe and operating a plunger of the microsyringe. As well known in the art, the microsyringe 14a is constructed of an elongated cylindrical syringe provided at one end thereof with a needle like an injection needle, and a plunger inserted into the syringe from the other end of the syringe (refer to FIG. 2). The plunger pump 14b comprises a base to which the syringe of the microsyringe 14a is fitted, and a movable element coupled to a base portion of the plunger of the microsyringe 14a and reciprocatively moved on the base along a longitudinal direction of the syringe of the microsyringe, and constructed so as to control a driving member such as a stepping motor by a controller having a suitable program written therein and operate the movable element at a predetermined speed.

In the specimen gas feeding mechanism 1, the specimen is injected into the inner tube from the automatic syringe 14 and fed from the inner tube 11 to the outer tube 12 by the aid of the oxygen and inert gas supplied through the carrier gas feed passage 51, and then while heating the outer tube 12 by the heating furnace 13, the specimen is oxidized and burned in a portion of the outer tube 12 located on the side of a tip end of the inner tube 11 by oxygen fed from the oxygen feed passage 52. The specimen gas feeding mechanism 1 is further constructed such that nitrogen in the specimen is converted into nitrogen monoxide and withdrawn as the specimen gas therefrom through the passage 61.

In the rear stage of the specimen gas feeding mechanism 1 (on the downstream side of a flowing direction of the specimen gas sampled), a dehydration bath 21 filled, for example, with phosphoric acid as a dehydration agent is disposed to subject the specimen gas to dehydration and scrubbing (washing) of gases. More specifically, the passage 61 extending from the outer tube 12 of the reaction tube 10 is connected to the dehydration bath 21. A gas outlet of the dehydration bath 21 is connected to the nitrogen analyzing mechanism 4 through the passage 62. Meanwhile, the dehydration bath 21 may be constructed of a fluororesin tube filled with fluororesin fibers which has a function of removing water.

The nitrogen analyzing mechanism 4 comprises an ozone generator 41 and a chemiluminescence detector 42, and is so designed as to measure an intensity of chemiluminescence generated by the reaction between nitrogen monoxide in the specimen gas fed from the specimen gas feeding mechanism 1 and ozone produced in the ozone generator 41 using the chemiluminescence detector 42. As the ozone generator 41, a generator utilizing a so-called ozonizer microelement which has, for example, such a structure that a D.C. voltage is applied between an anode and a cathode between which a solid polymer membrane is sandwiched and bonded to electrolyze water in air and thereby generate ozone at the anode, may be used from the standpoints of operability under a low voltage, reduction in size of the device, less occurrence of noise, prevention of generation of NOₓ, etc.

The chemiluminescence detector 42 is a detector of a reduced pressure chemiluminescence type in which light generated by the oxidation reaction is received by an electron multiplier tube and subjected to waveform processing to obtain an AREA value, and the amount of nitrogen in the specimen is measured from the AREA value using the below-mentioned previously prepared calibration curve. More specifically, in the nitrogen analysis using the chemiluminescence detector 42, nitrogen monoxide in the specimen gas is contacted with ozone to perform such an oxidation reaction as represented by NO + O₃ → NO₂ + O₂ + hN (wherein N is a frequency) to generate a light having a wavelength of 590 to 2500 nm. The thus generated light is received the electron multiplier tube to measure an intensity thereof, and subjected to the above processing.

The ozone generator 41 is connected with an oxygen feed passage 53 for introducing oxygen for production of ozone thereinto. The oxygen feed passage 53 may be a passage branched from the oxygen feed passage 52. The chemiluminescence detector 42 is connected with a passage 63 for feed of ozone extending from the ozone generator 41, and is also connected with a passage 62 extending from the aforementioned dehydration bath 21. Further, in a rear stage of the chemiluminescence detector 42, a detoxifying device 43 filled, for example, with activated carbon is disposed through a passage 64 to subject surplus ozone to detoxification treatment. In a rear stage of the detoxifying device 43, a vacuum pump 7 is disposed through a passage 65.

Meanwhile, although not shown in the drawings, the nitrogen analyzer may be constructed in the form of a multifunction analyzer capable of analyzing not only nitrogen but also chlorine and sulfur at the same time. In the multifunction analyzer, a chlorine analyzing mechanism and a sulfur analyzing mechanism are disposed at the position shown by reference numeral 3 in the course of the passage 62. As the chlorine analyzing mechanism, there may be used, for example, a mechanism comprising a titration cell filled with an electrolyte solution comprising acetic acid to subject hydrogen chloride in the specimen gas to coulometric titration in which the hydrogen chloride is absorbed into the acetic acid and titrated with silver ions coulometrically generated, to measure a quantity of electricity required for the titration and thereby compute an amount of chlorine. On the other hand, as the sulfur analyzing mechanism, there may be used a mechanism comprising an ultraviolet fluorescence detector for measuring a fluorescence intensity of sulfur dioxide in the specimen gas by irradiating the specimen gas with ultraviolet light in which the amount of sulfur in the specimen is measured using a calibration curve previously prepared, or a mechanism comprising a titration cell filled with an electrolyte solution comprising potassium iodide to subject sulfur dioxide in the specimen gas to coulometric titration in which the sulfur dioxide is absorbed into the aqueous solution of potassium iodide and titrated with triiodide ions coulometrically generated, to measure a quantity of electricity required for the titration and thereby compute an amount of sulfur, etc.

Next, the function of the computer for analysis used in the aforementioned nitrogen analyzer according to the present invention is explained. The analyzing process used in the nitrogen analyzer of the present invention is the same to the conventional analyzing process. Namely, the specimen comprising a nitrogen compound is burned to obtain a specimen gas, and the resulting specimen gas is reacted with ozone to measure a chemiluminescence intensity thereof, thereby quantitatively determining a concentration of nitrogen in the specimen.

Concretely, first, when injecting the specimen, the heating furnace 13 is energized to heat an inside of the reaction tube 10 to a temperature of 600 to 1100°C, and the vacuum pump 7 disposed on the downstream side is also operated. Next, in the specimen gas feeding mechanism 1, oxygen and an inert gas as a carrier gas are fed into the inner tube 11 through the carrier gas feed passage 51, and oxygen is fed to the outer tube 12 through the oxygen feed passage 52. Successively, the automatic syringe 14 is operated to inject a predetermined amount of the specimen, for example, fuel-related specimen, from the microsyringe 14a into the inner tube 11. The pressure and flow rate of the carrier gas and oxygen are adjusted to about 0.3 to 0.5 MPa and about 0.2 to 1 L/min, respectively, by controlling flow regulating valves (not shown) fitted to the feed passage 51 and the oxygen feed passage 52, respectively. With the above procedure, the specimen is oxidized in the outer tube 12 to convert nitrogen in the specimen into nitrogen monoxide, and the specimen gas comprising the nitrogen monoxide is withdrawn through the passage 61.

The specimen gas obtained in the reaction tube 10 is subjected to dehydration treatment in the dehydration bath 21, and then withdrawn through the passage 62 and introduced into the chemiluminescence detector 42 in the nitrogen analyzing mechanism 4. On the other hand, in the nitrogen analyzing mechanism 4, ozone is generated in the ozone generator 41, and introduced into the chemiluminescence detector 42 through the passage 63. In the chemiluminescence detector 42, the chemiluminescence intensity based on the reaction between nitrogen monoxide in the specimen gas and ozone is measured, and the amount of nitrogen in the specimen is calculated from the chemiluminescence intensity using the computer for analysis separately provided. Concretely, the amount of nitrogen in the specimen is calculated based on a calibration curve previously prepared with respect to a predetermined concentration range, and a whole nitrogen concentration in the specimen is quantitatively determined from the resulting value.

In the aforementioned nitrogen analyzing method, as the calibration curve, there is used a calibration curve previously prepared from a standard specimen having a nitrogen concentration of 5 to 100 ppm and preferably 5 to 60 ppm. Upon the analysis of the specimen, similarly to the standard specimen, the specimen is used in the form of a diluted specimen prepared by diluting the specimen with a solvent into a nitrogen concentration of 5 to 100 ppm and preferably 5 to 60 ppm.
By using the diluted specimen, it is possible to maintain a constant production efficiency of nitrogen monoxide upon burning the specimen in the reaction tube 10. Besides, in such a case, the weight of nitrogen in the diluted specimen is calculated using the calibration curve that is even more accurate in the nitrogen concentration range of 5 to 100 ppm in which a chemiluminescence area obtained from the chemiluminescence intensity as measured and the weight of nitrogen in the specimen are highly correlated with each other and have an approximately proportional relation with each other, so that it is possible to quantitatively determine the value of the nitrogen concentration with still higher accuracy.

The reason why the calibration curve prepared from a standard specimen having a nitrogen concentration of 5 to 100 ppm is used, and the diluted specimen having the same nitrogen concentration as that of the standard specimen is prepared, is as follows. That is, in the case of the specimen having a nitrogen concentration of more than 100 ppm, the relationship between the weight of nitrogen in the specimen and the chemiluminescence intensity (chemiluminescence area) corresponds to a correlation of linear equation in certain respective nitrogen concentration ranges. However, since the production efficiency of nitrogen monoxide upon burning is varied, the slopes of the linear equation in the respective nitrogen concentration ranges are different from each other and gradually decreased as the nitrogen concentration is increased, so that there is such a tendency that the measured value is gradually deviated from the true value. On the other hand, when it is intended to measure the specimen having a nitrogen concentration as low as less than 5 ppm, the detection sensitivity for the chemiluminescence intensity must be set to a higher value owing to problems concerning a sensitivity of a sensor used in the chemiluminescence detector 42.

In addition, in the present invention, in order to allow the concentration of nitrogen in the diluted specimen to fall within the above-specified range and accurately determine the amount of the diluted specimen, it is preferred to conduct the procedure of diluting the specimen using the aforementioned automatic syringe 14. More specifically, in the diluting procedure, by using the automatic syringe 14 capable of sucking the specimen into a syringe of the microsyringe 14a by mechanically operating a plunger of the microsyringe 14a, the specimen is diluted in the syringe of the microsyringe 14a.

For example, in the case where the specimen is a fuel-related specimen, as the solvent for diluting the specimen, there may be used an organic solvent. Specific examples of the solvent when using heavy oil, light oil, gasoline, etc., as the solvent, include toluene, xylene and trimethyl benzene. As shown in FIG. 2, when the specimen is diluted in the syringe of the microsyringe 14a using the above automatic syringe 14, for example, 10 to 20 *µ*L of a flush solvent (A), 0 to 5 *µ*L of air (preair) (B), 2 to 5 *µ*L of the specimen (C) and 30 to 50 *µ*L, of the solvent (D) are successively sucked into the syringe.

Upon preparing the diluted specimen, after injecting the specimen in a batch into the reaction tube 10, the flush solvent (A) is first sucked into the syringe to wash the microsyringe 14a. As the flush solvent (A), there may be used a liquid constituted of the same components as those of the solvent (D). However, in the case where the specimen (C) is water-soluble, water may be used as the flush solvent (A). The air (preair) (B) is sucked into the syringe for the purposes of completely driving the specimen (diluted specimen) out of the syringe of the microsyringe 14a and preventing inclusion of the flush solvent (A) into the specimen (C) and the solvent (D). Meanwhile, in the present invention, the amounts of the specimen (C) and the solvent (D) to be sucked and the dilution ratio may be optionally determined as far as the concentration of nitrogen in the diluted specimen is controlled to the above-specified range.

In the present invention, the relationship between the weight of nitrogen and chemiluminescence area in the aforementioned nitrogen concentration (calibration curve) is expressed by the equation: Y = aX + b (wherein X is a concentration of nitrogen monoxide or a weight of nitrogen in terms of nitrogen monoxide; Y is a chemiluminescence intensity; and a and b are each a constant coefficient) in which the value of the coefficient b (intercept) is approximately near zero. Meanwhile, the fact that the calibration curve obtained in the present invention forms a straight line of linear function can be confirmed by Mandel linearity test.

In the analysis using the above nitrogen analyzer, after the chemiluminescence intensity based on the reaction between nitrogen monoxide in the specimen gas and ozone is measured by the chemiluminescence detector 42, the chemiluminescence intensity (chemiluminescence area) is computed by the computer for analysis, and the weight of nitrogen in the diluted specimen is calculated based on the calibration curve previously written therein. Next, the concentration of nitrogen in the original specimen is calculated from the amount of the diluted specimen measured in the automatic syringe 14.

Meanwhile, in the case where the fuel-related specimen is analyzed in a refinery, etc., the specimen is diluted by referring to the nitrogen concentration thereof as measured in a consignor. However, the specimen whose nitrogen concentration is unknown is analyzed by a calibration curve separately prepared. In this case, if the nitrogen concentration (weight of nitrogen in the specimen) falls within the range of the calibration curve, the measuring result is adopted. On the other hand, in the case where the nitrogen concentration is higher than the maximum concentration of the calibration curve or in the case where the nitrogen concentration is lower than the minimum concentration of the calibration curve, an approximate value is calculated from the calibration curve, and the measurement is conducted again while varying the dilution ratio.

As described above, in the nitrogen analyzing method by using the diluted specimen prepared by diluting the specimen into a specific concentration range, it is possible to maintain a constant production efficiency of nitrogen monoxide upon burning the specimen, and further since the weight of nitrogen in the specimen can be calculated based on an accurate calibration curve in the aforementioned specific concentration range in which an approximately proportional relation between the nitrogen concentration and chemiluminescence peak area is established, it is possible to measure concentrations of nitrogen in various specimens that are extremely different in nitrogen concentration from each other, with high accuracy. In addition, in the nitrogen analyzing method, by using the automatic syringe 14 to dilute the specimen in the syringe, it is possible to exclude occurrence of human errors in the diluting procedure, and avoid exposure of human body to carcinogenic substances even when analyzing fuel-related specimens. Besides, according to the present invention, it is possible to conduct the analysis using a minimum amount of the diluted specimen and therefore further reduce environmental burden.

Also, as described above, the nitrogen analyzer according to the present invention, used for practicing the analyzing method, mainly comprises the specimen gas feeding mechanism 1, the nitrogen analyzing mechanism 4 and the computer for analysis. The specimen gas feeding mechanism 1 comprises the reaction tube 10 having a double tube structure comprising the inner tube 11 for introducing a liquid specimen which is provided at a head portion thereof with the automatic syringe 14 and the outer tube 12 for recovering the specimen gas through which oxygen is fed, and the heating furnace 13 for heating the reaction tube, and has a function of heating and burning the specimen in the reaction tube 10 by the heating furnace to convert nitrogen in the specimen into nitrogen monoxide and recover the nitrogen monoxide as the specimen gas. The nitrogen analyzing mechanism 4 comprises the ozone generator 41 and the chemiluminescence detector 42, and is constructed such that the chemiluminescence intensity based on the reaction between the nitrogen monoxide in the specimen gas fed from the specimen gas feeding mechanism 1 and ozone produced in the ozone generator 41 is measured by the chemiluminescence detector 42. When injecting the specimen into the specimen gas feeding mechanism 1, the diluting procedure in which the specimen is diluted with the solvent to prepare the diluted specimen having a nitrogen concentration of 5 to 100 ppm is conducted in the automatic syringe 14, and when quantitatively determining a concentration of nitrogen in the specimen from the chemiluminescence intensity obtained in the nitrogen analyzing mechanism 4, the computer for analysis calculates a weight of nitrogen in the diluted specimen based on a calibration curve previously prepared from a standard specimen having a nitrogen concentration of 5 to 100 ppm which expresses a relationship between the chemiluminescence intensity and the weight of nitrogen.

In consequence, according to the nitrogen analyzer, by preparing the diluted specimen by diluting the specimen into a specific concentration range in the automatic syringe 14, it is possible to maintain a constant production efficiency of nitrogen monoxide in the specimen gas feeding mechanism 1 upon burning the specimen, and further since the weight of nitrogen in the specimen can be calculated by the computer for analysis based on an accurate calibration curve in the aforementioned specific concentration range in which an approximately proportional relation between the nitrogen concentration and chemiluminescence intensity is established, it is possible to measure concentrations of nitrogen in various specimens that are extremely different in nitrogen concentration from each other, with high accuracy. In addition, by using the automatic syringe 14 to dilute the specimen in the syringe, it is possible to exclude occurrence of human errors in the diluting procedure, and avoid exposure of human body to carcinogenic substances even when analyzing fuel-related specimens. Besides, according to the present invention, it is possible to conduct the analysis using a minimum amount of the diluted specimen and therefore further reduce environmental burden.

### EXAMPLES

### [Example]

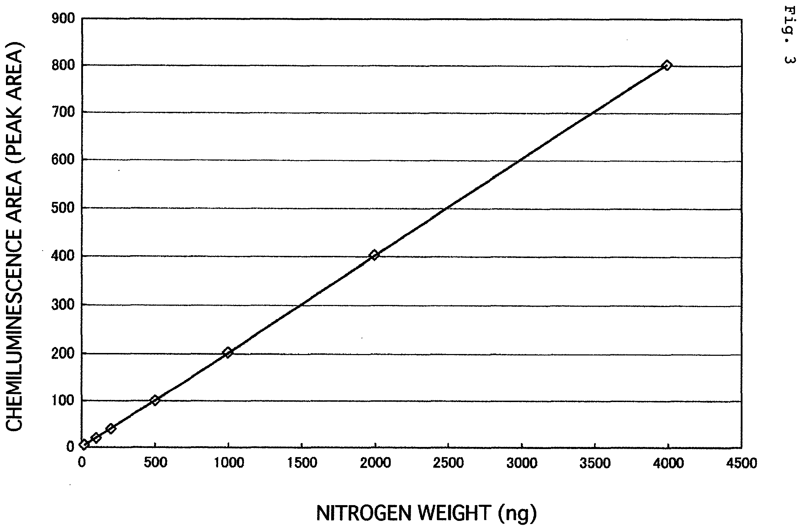

A plurality of specimens having known concentrations were prepared, and diluted with a solvent in the automatic syringe 14 of the nitrogen analyzer shown in FIG. 1. The obtained diluted specimens were analyzed by the nitrogen analyzer, thereby preparing a calibration curve. Various conditions used in Example are as follows, and the thus prepared calibration curve is as shown in FIG. 3.

**[Table 1]**

| | |
|---|---|
| Nitrogen standard substance (specimen): pyridine; | |
| Solvent: xylene; | |
| Concentration of nitrogen in specimen: 10 ppm, 50 ppm, 100 ppm, 250 ppm, 1000 ppm and 2000 ppm; | |
| Diluting procedure: | |
| | Flush solvent: water, 10 *µ*L; |
| | Air (preair): 2 *µ*L; |
| | Amount of specimen sucked: 2 *µ*L; |
| | Solvent: 38 *µ*L (dilution ratio: 20 times); |
| Burning conditions: | |
| | Inlet temperature: 800°C; |
| | Outlet temperature: 900°C; |
| Calibration curve prepared: | |
| | Linear regression: y = 0.2008x - 0.1066 (R² = 1) |
| | Quadratic regression: y = -3E-07x² + 0.202x - 0.4032 (R² = 1) |

### [Comparative Example]

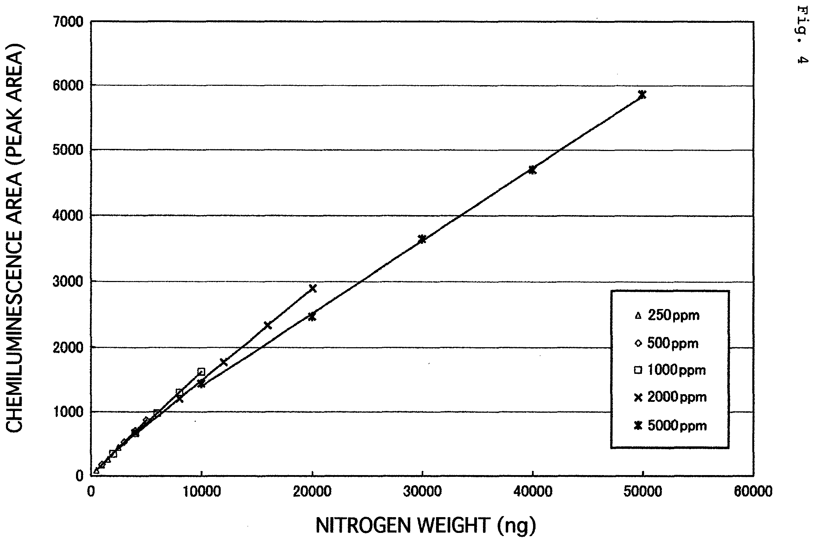

A plurality of specimens having known concentrations were prepared, and respectively analyzed by the nitrogen analyzer shown in FIG. 1, thereby preparing a calibration curve. The procedure of Comparative Example was different from that of Example in such points that the concentrations of nitrogen in the specimens as well as the kinds of solvents used were different from those of Example, and the specimens were not diluted. The diluting conditions used in Comparative Example are as follows, and the thus prepared calibration curves are as shown in FIG. 4.

**[Table 2]**

| | |
|---|---|
| Nitrogen standard substance (specimen): pyridine; | |
| Solvent: toluene; | |
| Concentration of nitrogen in specimen: 250 ppm, 500 ppm, 1000 ppm, 2000 ppm and 5000 ppm; | |
| Amount of specimen injected: 2 *µ*L, 4 *µ*L, 6 *µ*L, 8 *µ*L and 10 *µ*L; | |
| Burning conditions: | |
| | Inlet temperature: 800°C; |
| | Outlet temperature: 900°C; |
| Calibration curve at a nitrogen concentration of 250 ppm: | |
| | Linear regression: y = 0.1808x - 0.8573 (R² = 1) |
| | Quadratic regression: y = 8E-07x² + 0.1784x - 0.5433 (R² = 1) |
| Calibration curve at a nitrogen concentration of 500 ppm: | |
| | Linear regression: y = 0.1751x + 2.9147 (R² = 1) |
| | Quadratic regression: y = 2E-07x² + 0.1741x + 4.1559 (R² = 1) |
| Calibration curve at a nitrogen concentration of 1000 ppm: | |
| | Linear regression: y = 0.1611x + 23.608 (R² = 1) |
| | Quadratic regression: y = 1E-07x² + 0.1597x + 26.875 (R² = 1) |
| Calibration curve at a nitrogen concentration of 2000 ppm: | |
| | Linear regression: y = 0.1405x + 90.024 (R² = 0.9999) |
| | Quadratic regression: y = 1E-07x² + 0.1378x + 102.57 (R² = 1) |
| Calibration curve at a nitrogen concentration of 5000 ppm: | |
| | Linear regression: y = 0.1105x + 308.9 (R² = 0.9995) |
| | Quadratic regression: y = 1E-07x² + 0.1043x + 381.12 (R² = 0.9997) |

As shown in FIG. 4, the calibration curves prepared by the conventional methods have high linearity in the respective nitrogen weight ranges. However, the calibration curves had such a tendency that as the nitrogen concentration was increased, the slope of the respective curves became smaller, and the intercept thereof is more largely spaced apart from the origin in the positive direction. As a result, it was confirmed that as the nitrogen concentration was varied, the production efficiency of nitrogen monoxide by burning was changed. On the other hand, in the present invention, as shown in FIG. 3, the calibration curve capable of maintaining a high proportional relation was obtained.

### INDUSTRIAL APPLICABILITY

In the nitrogen analyzer according to the present invention, there is used the diluted specimen prepared by diluting the specimen into the specific concentration range in which an approximately proportional relationship between the nitrogen concentration and chemiluminescence peak area is established, so that it is possible to measure concentrations of nitrogen in various specimens that are extremely different in nitrogen concentration from each other, with high accuracy. The nitrogen analyzer according to the present invention can be suitably used for quantitative analysis of nitrogen in fuel-related specimens including petroleum such as diesel fuel, oils and gasoline, and coals such as coal tar.

### Reference Signs List

1: Sample gas feeding mechanism; 10: Reaction tube; 11: Inner tube; 12: Outer tube; 13: Heating furnace; 14: Automatic syringe; 14a: Microsyringe; 14b: Plunger pump; 4: Nitrogen analyzing mechanism; 41: Ozone generator; 42: Chemiluminescence detector; A: Flush solvent; B: Air (preair); C: Specimen; and D: Solvent.

## Claims

1. A nitrogen analyzer comprising a specimen gas feeding mechanism (1), a nitrogen analyzing mechanism (4) and a computer for analysis,
wherein the specimen gas feeding mechanism (1) comprises a reaction tube (10) having a double tube structure comprising an inner tube (11) for introducing a liquid specimen, which comprises at a head portion thereof a syringe (14), and an outer tube (12) for recovering a specimen gas, the outer tube (12) being configured for feeding oxygen therethrough, and a heating furnace (13) for heating the reaction tube (10), the specimen gas feeding mechanism (1) having a function of heating and burning the specimen in the reaction tube (10) by the heating furnace (13) to convert nitrogen in the specimen into nitrogen monoxide and recover the nitrogen monoxide as the specimen gas;
wherein the nitrogen analyzing mechanism (4) comprises an ozone generator (41) and a chemiluminescence detector (42), the chemiluminescence detector (42) being constructed to measure a chemiluminescence intensity based on a reaction between the nitrogen monoxide in the specimen gas fed from the specimen gas feeding mechanism (1) and ozone produced in the ozone generator (41), and wherein in use, when injecting a predefined amount of the specimen into the specimen gas feeding mechanism (1), the nitrogen analyzer is configured to perform a diluting procedure in which the specimen is diluted with a solvent to prepare a diluted specimen having a nitrogen concentration of 5 to 100 ppm being conducted in the syringe (14);
**characterized in that**
the syringe (14) is an automatic syringe,
and when quantitatively determining a concentration of nitrogen in the specimen from the chemiluminescence intensity obtained in the nitrogen analyzing mechanism (4), the computer for analysis comprises means for calculating a weight of nitrogen in the diluted specimen based on a calibration curve previously prepared from a standard specimen having a nitrogen concentration of 5 to 100 ppm which expresses a relationship between a chemiluminescence peak area obtained from the chemiluminescence intensity as measured and the weight of nitrogen.

2. The nitrogen analyzer according to claim 1, wherein the nitrogen analyzer is configured to be used with the specimen being a fuel-related specimen, and the solvent being an organic solvent.

3. The nitrogen analyzer according to claim 2, wherein the nitrogen analyzer is configured to be used with the organic solvent being toluene, xylene or trimethyl benzene.

4. The nitrogen analyzer according to any one of claims 1 to 3, wherein for preparing the diluted specimen, the nitrogen analyzer is configured to sequentially suck 10 to 20 µL of a flush solution, 0 to 5 µL of air, 2 to 5 µL of the specimen and 30 to 50 µL of the solvent into the syringe (14a).

5. The nitrogen analyzer according to claim 4, wherein the nitrogen analyzer is configured to be used with the flush solution being a liquid comprising the same components as those of the solvent or water.

## Patentansprüche

1. Stickstoffanalysator, umfassend einen Probegas-Zufuhrmechanismus (1), einen Stickstoffanalysemechanismus (4) und einen Computer zur Analyse,
wobei der Probegas-Zufuhrmechanismus (1) ein Reaktionsrohr (10) umfasst, das eine Doppelrohrstruktur aufweist, die ein Innenrohr (11) zum Einführen einer flüssigen Probe umfasst, welches an einem Kopfabschnitt davon eine Spritze (14) umfasst, und ein Außenrohr (12) zur Gewinnung eines Probegases, wobei das Außenrohr (12) dazu konfiguriert ist, Sauerstoff hindurch zu fördern, und einen Heizofen (13) zum Heizen des Reaktionsrohrs (10), wobei der Probegas-Zufuhrmechanismus (1) eine Funktion zum Erhitzen und Verbrennen der Probe in dem Reaktionsrohr (10) mittels des Heizofens (13) aufweist, um Stickstoff in der Probe in Stickstoffmonoxid umzuwandeln und das Stickstoffmonoxid als das Probegas zu gewinnen;
wobei der Stickstoffanalysemechanismus (4) einen Ozongenerator (41) und einen Chemolumineszenz-Detektor (42) umfasst, wobei der Chemolumineszenz-Detektor (42) dazu konstruiert ist, eine Chemolumineszenz-Intensität basierend auf einer Reaktion zwischen dem Stickstoffmonoxid in dem von dem Probegas-Zufuhrmechanismus (1) zugeführten Probegas und dem in dem Ozongenerator (41) erzeugten Ozon zu messen, und
wobei bei der Verwendung, beim Einspritzen einer vordefinierten Menge von der Probe in den Probegas-Zufuhrmechanismus (1), der Stickstoffanalysator dazu konfiguriert ist, ein Verdünnungsverfahren durchzuführen, das in der Spritze (14) durchgeführt wird, bei welchem die Probe mit einem Lösungsmittel verdünnt wird, um eine verdünnte Probe herzustellen, die eine Stickstoffkonzentration von 5 bis 100 ppm aufweist;
**dadurch gekennzeichnet, dass**
die Spritze (14) eine automatische Spritze ist,
und beim quantitativen Bestimmen einer Konzentration von Stickstoff in der Probe aus der in dem Stickstoffanalysemechanismus (4) erhaltenen Chemolumineszenz-Intensität, der Computer zur Analyse Mittel zum Berechnen eines Stickstoffgewichts in der verdünnten Probe umfasst, basierend auf einer Kalibrierungskurve, die zuvor von einer Standardprobe mit einer Stickstoffkonzentration von 5 bis 100 ppm erstellt worden ist, welche eine Beziehung zwischen einem aus der gemessenen Chemolumineszenz-Intensität erhaltenen Chemolumineszenz-Peak-Bereich und dem Stickstoffgewicht ausdrückt.

2. Stickstoffanalysator nach Anspruch 1, wobei der Stickstoffanalysator dazu konfiguriert ist, mit der Probe verwendet zu werden, die eine Brennstoffprobe ist, und dem Lösungsmittel, das ein organisches Lösungsmittel ist.

3. Stickstoffanalysator nach Anspruch 2, wobei der Stickstoffanalysator dazu konfiguriert ist, mit dem organischen Lösungsmittel verwendet zu werden, das Toluol, Xylol oder Trimethylbenzol ist.

4. Stickstoffanalysator nach einem der Ansprüche 1 bis 3, wobei der Stickstoffanalysator zur Herstellung der verdünnten Probe dazu konfiguriert ist, nacheinander 10 bis 20 µl einer Spüllösung, 0 bis 5 µl Luft, 2 bis 5 µl der Probe und 30 bis 50 µl des Lösungsmittels in die Spritze (14a) einzusaugen.

5. Stickstoffanalysator nach Anspruch 4, wobei der Stickstoffanalysator dazu konfiguriert ist, mit der Spüllösung verwendet zu werden, die eine Flüssigkeit ist, welche die gleichen Komponenten umfasst wie die des Lösungsmittels oder Wasser.

## Revendications

1. Analyseur d'azote comprenant un mécanisme d'alimentation en gaz d'échantillon (1), un mécanisme d'analyse d'azote (4) et un ordinateur pour une analyse,
dans lequel le mécanisme d'alimentation en gaz d'échantillon (1) comprend un tube réactionnel (10) ayant une structure à double tube comprenant un tube interne (11) pour introduire un échantillon liquide, qui comprend au niveau d'une partie supérieure de celui-ci une seringue (14), et un tube externe (12) pour récupérer un gaz d'échantillon, le tube externe (12) étant configuré pour alimenter de l'oxygène à travers celui-ci, et un four de chauffage (13) pour chauffer le tube réactionnel (10), le mécanisme d'alimentation en gaz d'échantillon (1) ayant une fonction de chauffage et de combustion de l'échantillon dans le tube réactionnel (10) via le four de chauffage (13) pour convertir l'azote dans l'échantillon en monoxyde d'azote et récupérer le monoxyde d'azote en tant que gaz d'échantillon ;
dans lequel le mécanisme d'analyse d'azote (4) comprend un générateur d'ozone (41) et un détecteur de chimioluminescence (42), le détecteur de chimioluminescence (42) étant conçu pour mesurer une intensité de chimioluminescence basée sur une réaction entre le monoxyde d'azote et le gaz d'échantillon alimenté par le mécanisme d'alimentation en gaz d'échantillon (1) et l'ozone produit dans le générateur d'ozone (41), et
dans lequel, en fonctionnement, lors de l'injection d'une quantité prédéfinie de l'échantillon dans le mécanisme d'alimentation en gaz d'échantillon (1), l'analyseur d'azote est configuré pour effectuer une procédure de dilution dans laquelle l'échantillon est dilué avec un solvant pour préparer un échantillon dilué ayant une concentration en azote de 5 à 100 ppm conduite dans la seringue (14) ;
**caractérisé en ce que**
la seringue (14) est une seringue automatique et lorsqu'il est déterminé quantitativement une concentration d'azote dans l'échantillon à partir de l'intensité de chimioluminescence obtenue dans le mécanisme d'analyse d'azote (4), l'ordinateur d'analyse comprend des moyens pour calculer un poids d'azote dans l'échantillon dilué sur la base d'une courbe d'étalonnage préparée au préalable à partir d'un échantillon standard ayant une concentration en azote de 5 à 100 ppm exprimant une relation entre une aire de pic de chimioluminescence obtenue à partir de l'intensité de chimioluminescence mesurée et le poids d'azote.

2. Analyseur d'azote selon la revendication 1, dans lequel l'analyseur d'azote est configuré pour être utilisé avec l'échantillon qui est un échantillon relatif à un carburant et le solvant qui est un solvant organique.

3. Analyseur d'azote selon la revendication 2, dans lequel l'analyseur d'azote est configuré pour être utilisé avec le solvant organique qui est du toluène, du xylène ou du triméthylbenzène.

4. Analyseur d'azote selon l'une quelconque des revendications 1 à 3, dans lequel pour préparer l'échantillon dilué, l'analyseur d'azote est configuré pour aspirer séquentiellement 10 à 20 µL d'une solution de rinçage, 0 à 5 µL d'air, 2 à 5 µL de l'échantillon et de 30 à 50 µL du solvant dans la seringue (14a).

5. Analyseur d'azote selon la revendication 4, dans lequel l'analyseur d'azote est configuré pour être utilisé avec la solution de rinçage qui est un liquide comprenant les mêmes composants que ceux du solvant ou de l'eau.
